# EUROPÄISCHE PATENTANMELDUNG

(11) **EP 1 310 261 A1**
(43) Veröffentlichungstag der Anmeldung: **14.05.2003**
(21) Anmeldenummer: 01402887.2
(22) Anmeldetag: 09.11.2001
(51) Int. Cl.: A61K 35/78, A61K 7/48, A61P 17/00

(54) **Verwendung eines Extraktes der Vigna aconitifolia-Pflanze in einer Kosmetischen und/oder dermopharmazeutischen Zusammensetzung**

(71) Anmelder: Cognis France S.A., 31360 Saint-Martory (FR)
(72) Erfinder: Contet-Audonneau, Jean-Luc, 54130 Saint-Max (FR); Danoux, Louis, 54420 Saulxures-Les-Nancy (FR); Pauly, Gilles, 54000 Nancy (FR); Moser, Philippe, 54270 Essey-Les-Nancy (FR)
(74) Vertreter: Cabinet HERRBURGER

(57) **Zusammenfassung**

Verwendung eines proteinhaltigen Extraktes der Vigna aconitifolia-Pflanze in einer kosmetischen oder dermopharmazeutischen Zusammensetzung.

Die Zielsetzung der vorliegenden Erfindung besteht in der Verwendung als Wirkstoff in einer kosmetischen oder dermopharmazeutischen Zusammensetzung für die örtlich wirkende Anwendung auf der Haut, den Epithelanhanggebilden und/oder den Schleimhäuten von mindestens einer lösbaren Proteinfraktion, die aus dem Samen der Vigna aconitifolia-Pflanze extrahiert wurde.

Ein weiterer Gegenstand der Erfindung sind kosmetische und/oder dermopharmazeutische Zubereitungen, enthaltend einen proteinhaltigen Extrakt der Vigna acnitifolia.

## Beschreibung

### Gebiet der Erfindung

Die vorliegende Erfindung bezieht sich auf das Gebiet der Kosmetik und der Dermopharmazie. Ihre Zielsetzung besteht in der Verwendung von mindestens einem proteinhaltigen Extrakt der Vigna aconitifolia-Pflanze. Ein weiterer Gegenstand der Erfindung sind kosmetische und/oder dermopharmazeutische Zubereitungen, die einen derartigen Extrakt enthalten.

### Stand der Technik

Auf der Suche nach neuen Nahrungsquellen für Entwicklungsländer wurde man aufmerksam auf die in Sri Lanka, im Himalaya, Burma, Sudan und Ostafrika kultivierten Formen der anspruchslosen und trockenresistenten Samen der Vigna aconitifolia [Jacq.] Marechal (Fabaceae), Mücken-oder Mottenbohne. Während die gekochten trockenen Samen der Vigna aconitifolia eine geringe Nahrungsqualität haben, werden die Hülsen verbreitet als Nahrungsmittel verwendet.

Der Gehalt der Samen beträgt durchschnittlich 61,9 % Kohlenhydrate, 21,9 % Proteine und 3,48 % Lipide, sowie 1,3 % Polyphenole und 0,65 % Phytinsäure (phytic acid).
In einer Übersicht von Kadam et Salunke [S.S. Kadam, D.K. Salunkhe, Nutritional composition, processing and utilization of horse gram and moth bean, CRC Critical reviews in Food Science and Nutrition, 1985, 1-26] wird über die Anwesenheit weiterer Inhaltsstoffe wie Trypsin Inhibitoren, Hemagglutinine und alpha-Amylase-Inhibitoren berichtet.

Während bei anderen weniger bekannten Vigna-Arten eine pflegende und heilende Wirkung der Samenextrakte bereits aus der französischen Patentanmeldung FR 2796839 A1 für die Vigna trilobata bekannt wurde, konnte den Samen der Vigna aconitifolia bisher lediglich eine dietätisch aufbauende Wirkung bei Fieberpatienten zugeschrieben werden.

In dem US-Patent US 5 322 839 werden Proteinfraktionen der Samen von Leguminosen, insbesondere Soya- und Limabohnen als Wirkstoffe mit antientzündlichen, Elastase inhibierenden und Trypsin inhibierenden Eigenschaften offenbart.

Zunehmend wird jedoch auf dem kosmetischen und pharmazeutischen Markt nach pflanzlichen Wirkstoffen gesucht, die für Haut beispielsweise pflegende, vor Alterserscheinungen schützende, und revitalisierende Eigenschaften vermitteln. Die Zusammensetzung des Produktes soll zusätzlich eine optimale dermatologische Verträglichkeit besitzen, so dass auch empfindliche Verbraucher nicht mit Irritationen reagieren. Darüber hinaus sollten die Mittel jedoch auch weitere Funktionen erfüllen, die gleichzeitig die technischen Eigenschaften des kosmetischen Produktes, wie Lagerstabilität, Lichtstabilität und Formulierbarkeit positiv beeinflussen oder zumindest nicht verschlechtern.

Die komplexe Aufgabe der vorliegenden Patentanmeldung hat darin bestanden, neue Wirkungen von bereits bekannten Pflanzen zu finden und die Verwendung dieser Extrakte in kosmetischen und/oder dermopharmazeutischen Mitteln zu ermöglichen, die sich durch eine hohe Verträglichkeit auch für empfindliche Haut auszeichnen und zusätzlich eine gute physiko-chemische Stabilität aufweisen.

### Beschreibung der Erfindung

Gegenstand der vorliegenden Erfindung ist die Verwendung eines aus der Pflanze Vigna aconitifolia extrahierten proteinhaltigen Extraktes zur Herstellung kosmetischer und/oder dermopharmazeutischer Zubereitungen für die lokale Anwendung auf der Haut, den Epithelanhanggebilden und/oder den Schleimhäuten.
Ein weiterer Gegenstand der Erfindung sind kosmetische und/oder dermopharmazeutische Zubereitungen für die lokale Applikation, enthaltend einen proteinhaltigen Extrakt der Pflanze Vigna aconitifolia.

Es wurde überraschender Weise festgestellt, dass die Proteinextrakte der Vigna aconitifolia bei lokaler Anwendung auf der Haut, den Epithelanhanggebilden und den Schleimhäuten besondere biologische Wirkungen hervorrufen und über eine sehr gute Toleranz verfügen und dass die Verwendung als Wirkstoff, alleine oder in Verbindung mit mindestens einem anderen Wirkstoff, von mindestens einer aus Vigna aconitifolia extrahierten Proteinfraktion in einer Zusammensetzung oder einem kosmetischen oder dermopharmazeutischen Erzeugnis bei lokaler Anwendung auf der Haut, den Epithelanhanggebilden und/oder den Schleimhäuten es ermöglicht, regenerative Wirkungen, Anti-Reizwirkungen, Anti-Aging Effekte und wachstumsfördernde Aktivitäten festzustellen.
Die proteinhaltigen Extrakte der Vigna aconitifolia zeichnen sich besonders zur vorbeugenden und heilenden Behandlung empfindlicher Hauttypen aus. Sie werden hervorragend vertragen. Im Human in vivo Versuch zeigt sich, dass schon bei geringer Dosis an Vigna aconitifolia Extrakten in den lokal applizierten Zubereitungen eine deutlich erhöhte Zellerneuerungsrate der menschlichen Epidermis beobachtet werden kann.
Besonders die Proteinfraktionen aus dem Samen der Vigna aconitifolia zeigen Eigenschaften wie ein ausgeprägte Stimulationswirkungen des Zellenwachstums und -metabolismus (energiespendende, stimulierende, Anti-Älterungs-Aktivität), starke Anti-Apoptose-Wirkung, eine im Rahmen der Apoptosewirkung schützende Aktivität gegen oxidativen Stress und eine Beschleunigung des epidermalen Zellerneuerungscyclus.
Als Wirkstoff im Sinne der vorliegenden Erfindung werden Extrakte der Pflanze Vigna aconitifolia, insbesondere Extrakte der Samen von Vigna aconitifolia verstanden. Dabei sind ausgeprägte Wirkungen besonders den Proteinfraktionen der Pflanzenextrakte und bevorzugt Proteinfraktionen aus den Samen der Vigna aconitifolia zuzuschreiben. Bei den extrahierten Fraktionen, die den Wirkstoff bilden, werden Zusammensetzungen die mindestens zwei Proteinfraktionen enthalten oder die mindestens eine lösliche Proteinfraktion beinhalten speziell bevorzugt.

Die proteinhaltigen Extrakte lassen sich in Kombination mit weiteren Hilfsstoffen wie Polyole, Antioxidantien und Konservierungsmittel leicht in herkömmliche Formulierungen einarbeiten, die dann eine gute physiko-chemische Stabilität aufweisen.
Die Extrakte oder lösbaren Proteinfraktionen nach der Erfindung können auch in jeden anderen zutreffenden kosmetischen Vektor eingearbeitet werden oder mit ihm verbunden werden, zum Beispiel filmbildende Mittel, Liposome, Zyklodextrine, Micellen, Makro-, Mikro- und Nanopartikel, sowie Makro-, Mikro und Nanokapseln oder sie können absorbiert oder auf organische Polymere oder mineralische Träger verpflanzt werden.

### Beispiele für den Nachweis der Eigenschaften der Proteinextrakte von Vigna aconitifolia

Die biologischen Eigenschaften und Aktivitäten der Proteinextrakte von Vigna aconitifolia konnten durch Tests bestimmt und gemessen werden, die dem Fachmann auf dem Gebiet bekannt sind, ihre Ergebnisse werden nachstehend aufgeführt:

### 1. Zellwachstums und Überlebens - Test

Für die Beurteilung der Zellaktivität gibt es wesentliche Marker, zu denen ATP, Proteine und Glutathion zählen.
ATP (Adenosintriphosphat) ist ein Zellbestandteil, der Energie speichert und vorwiegend in Mitochondrien produziert wird. Zellen benötigen ATP, um die Aktivität ihrer Enzyme zu sichern, die wiederum das Cytoskellet, die lonenkanäle, die Nahrungsaufnahme und viele lebenswichtige biologische Prozesse der Zelle steuern.
(Vasseur P., Aerts C. Appréciation de la cytotoxicité par la mesure de l'ATP. Journal français Hydrologie (1982) vol 9, pp 149-156).
Die Proteinkonzentration der Zellen wurde bestimmt nach Bradford (Bradford M.M. A rapid and sensitive method for the quantitation of microgram quantities of protein utilizing the principle of protein-dye binding. Anal. Biochem. (1977) vol 72, pp 248-254)
Glutathion (GSH) ist ein Peptid, das von Zellen produziert wird, um die Zelle vor oxidativem Stress oder Schwermetallen wie beispielsweise Blei oder Quecksilber zu schützen. Die drei Aminosäuren die bei der reduzierten Form von GSH involviert sind, sind wiederum verbunden mit spezifischen cytoplasmatischen Enzymen, die ATP benötigen.
Die Steigerung des GSH-Levels hat einen positiven Einfluß auf die Aktivität der Glutathion-Stransferase, die ein entgiftendes Enzym darstellt.
GSH wurde bestimmt nach Hissin (Hissin P.J., Hilf R. A fluorometric method for determination of oxidised and reduced Glutathione in tissues. Analytical Biochemistry (1977) vol 74, pp 214-226).

### 1a) WIRKUNGEN AUF DAS ZELLENWACHSTUM (Tabelle 1a)

Menschliche Fibroblaste werden in ein Nährmedium (DMEM = Dulbecco Minimum Essential Medium von der Firma Life Technologie Sarl) mit 10 % fötalem Kälberserum (von der Firma Dutcher) geimpft und 24 Stunden lang bei 37° C in einer 5 %igen CO₂-Athmosphäre inkubiert.

Das Wachstumsmedium wird danach durch ein Sub-Optimum-Medium (ohne SVF) ersetzt, das verschiedene Konzentrationen von Extrakten (0 ; 0,1 und 0,3 % Gew.Nol., Gewicht/Volumen) nach der Beschreibung der Erfindung enthält. Nach einer 3tägigen Inkubation bei 37°C wurde das Wachstum durch eine Zählung der haftenden Zellen durch einen automatischen Partikelzähler und durch die Dosierung der intrazelluläre ATP-Gehalt ausgewertet.

### 1b) WIRKUNGEN AUF DAS ÜBERLEBEN (Tabelle 1b)

Der Test wird auf Fibroblasten nach dem gleichen Protokoll wie das Wachstum vorgenommen, wobei die Inkubationsdauer wiederum 72 Stunden bei 37°C beträgt.

Das Überleben wurde durch die Dosierung der folgenden Gehalte ausgewertet:
- Rate des metabolisierten MTT (Methyl Thiazolyl Tetrazolium)
   Die Aktivität der Mitochondrien wird über den MTT-Test bestimmt. MTT wird durch ein Enzym der Respirationskette, Succinatdehydrogenase, in Formazan reduziert (Denizot F, Lang R, Rapid colorimetric assay for cell growth and survival. J. Immunol. Methods, 89, 271-277, 1986).
- von Proteinen,
- von Glutathion (GSH), ein direkt von der Zelle erzeugtes Peptid, zur Bekämpfung von oxydativem Stress oder von verschiedenen Schmutzstoffen, wie zum Beispiel Schwermetalle. Seine Synthese erfordert ATP als Energiequelle.

Die Versuche 1a und 1b wurden in dreifacher Ausführung vorgenommen und zwei- oder dreimal wiederholt. Die in unterschiedlichen Konzentrationen eingesetzten Extrakte der Vigna aconitifolia wurden hergestellt nach dem Verfahren Beispiel 7.
Die Ergebnisse werden im Verhältnis zu einer extraktfreien Formulierung für Protein, ATP, MTT und GSH in das Verhältnis gestellt und in einem Prozentsatz im Verhältnis zum unbehandelten Kontrollmittel angegeben als Durchschnittswert +/- SEM (Fehlertyp des Durchschnitts) ausgedrückt.

**Table 1a:**

| **Wachstum- Test** Ergebnisse in % basierend auf der Kontrolle ohne Extrakt (Mittelwert von 2 Assays in dreifacher Ausführung) | | |
|---|---|---|
| Konzentration Extrakt (% Gew./Vol.) | Proteine | ATP |
| 0 | 100 | 100 |
| 0.1 | 107 | 113 |
| 0.3 | 110 | 114 |

Die Ergebnisse in Tabelle 1a zeigen, dass die Extrakte bei Dosen von 0,1 % (Gew./Vol. - Gewicht-Volumen) die Anzahl der Zellen und die ATP-Gehalt in den menschlichen Fibroblasten in in vitro-Wachstum deutlich steigern. Die.Werte stellen jeweils die nach drei Tagen gezählten Zellen und die ATP-Gehalt nach drei Tagen für verschiedene Konzentrationen dar.

**Table 1b:**

| **Zellüberlebens - Test** Ergebnisse in % basierend auf der Kontrolle ohne Extrakt (Mittelwert von 3 Assays in dreifacher Ausführung) | | | |
|---|---|---|---|
| Konzentration des Extraktes % Gew./Vol. | MTT | Proteine | GSH / Proteine |
| 0 | 100 | 100 | 100 |
| 0.1 | 100 | 107 | 101 |
| 1 | 111 | 118 | 122 |

Die Tabelle 1b gibt jeweils die Mitochondrienaktivität über die MTT, Proteingehalte und die GSH-Gehalte an, die nach drei Tagen für verschiedene Konzentrationen an Extrakten (0,1 und 1 % Gew./Vol.) gemessen wurden. Ein Extrakt der Vigna aconitifolia mit einer Konzentration von 1 Gew.% hat die Mitochondrienaktivität danach erheblich erhöht (+ 11%) Die Ergebnisse zeigen, dass die Proteinextrakte von Vigna aconitifolia schon bei 0,1 % (Gew.Nol.) den Gehalt an synthetisierten Proteinen deutlich steigern (+ 7%). Das Glutathion in den menschlichen Fibroblasten ist nach Behandlung mit 1 %igem Extrakt ebenfalls deutlich erhöht.

Diese Ergebnisse zeigen, dass die ursprünglichen oder hydrolysierten Proteinextrakte von Vigna aconitifolia hohe Fähigkeiten zur Verbesserung des Wachstums und des Metabolismus (Synthese von ATP, der Proteine und des Glutathion) durch die menschlichen Fibroblaste aufweisen, was deutlich eine energiespendende, stimulierende und "Anti-Älterungs"- Aktivität dieser Extrakte angibt.

### 2. Test zur Anti-Apoptose-Aktivität

Die Apoptose ist ein biologischer, aktiver Vorgang, der von lebenden Organismen verwendet wird, um gewisse Zellen ihrer Gewebe durch Autolyse zu beseitigen, insbesondere eine Zerstörung der Proteine und der nuklearen DNA in kleine Fragmente, die in das Zytoplasma ausgesalzen werden.
Die Apoptose kann auch durch einen oxydativen Stress (UV-R, Entzündung), durch eine Entbehrung von Wachstumsfaktoren oder durch giftige Substanzen (Schadstoffe, genotoxische Stoffe ...) induziert werden.
Durch Entzug der entsprechenden Faktoren, die Überlebensbotschaften an die Zellen senden, kann das Selbstmordprogramm gestartet und die Apoptose ausgelöst werden. Eine anschließende Bestimmung der Zellzahlen erlaubt Aufschluß über den Umfang des ausgelösten Zelltods. Das System ist damit bestens geeignet, die Wirkung von Anti-Apoptosewirkstoffen zu verfolgen. Dabei kann das Verfahren sowohl auf ex-vivo- als auch auf in-vitro-Haut-kulturen (Fibroblasten, Keratinocyten, Epithelzellen) sowie auf ex-vivo-Kulturen von menschlichen Haarfollikeln angewendet werden.

### Nachweis der Apoptose und Bestimmung des Gehaltes an apoptotischen Zellen - in-vitro

Im Verlauf der Apoptose werden durch den Angriff von Endonucleasen Fragmente von den im Zellkern enthaltenen DNA-Strängen abgespalten und in das Cytoplasma entlassen. Wie beispielsweise von Henseleitet al. in **Archiv.Derma-tol.Res. 288(11), 676 (1996)** oder Parat et al. in **J.Photochem.Photobiol B.Biol. 37, 101 (1997)** beschrieben, kann der Apoptose-level z.B. nach der ELISA-Methode bestimmt werden.

Untersucht wurde die Fähigkeit der Pflanzenextrakte der Vigna aconitifolia, die durch einen Mangel an Wachstumsfaktoren induzierte Apoptose in humanen Hautzellen zu verhindern. An humanen Fibroblasten(Versuch 2a) und humanen Keratinocyten (Versuch 2b) wurden diese Test in vitro durchgeführt. Die humanen Zellen wurden in einem Nährmedium (DMEM = Dulbecco Minimum Essential Medium von der Firma Life Technologie Sarl) mit 10 % fötalem Kälberserum (FCS= fötal calf serum, von der Firma Dutcher) kultiviert. Diesem Nährmedium wurde Bromodesoxyuridin (BrdU) zugegeben, welches in die DNA eingebaut wurde und später zum Nachweis der DNA-Fragmente in dem Cytoplasma diente. Nach 3 Tagen Inkubation erhalten die Zellen dann ein Überlebensmedium (DMEM) ohne Serum oder Wachstumsfaktoren, das verschiedene Konzentrationen (0 ; 0,05 ; 0,1 % Gew./Vol.) der zu testenden Extrakte der Vigna aconitifolia enthält und werden 2 Tage lang bei 37° C inkubiert. Nach der Inkubation werden die Zellen durch Trypsinierung zurückgewonnen und danach analysiert.

### Mengenbestimmung der Gehalt der apoptotischen Zellen

In dieser Methode werden die Zellen lysiert (nicht der Zellkern) und der Gehalt der apoptotischen Zellen wird durch einen ELISA-Test mengenbestimmt, der BrdU offenbart, das in die zytoplasmischen ADN-Fragmente eingebaut wurde.

Die Ergebnisse werden in einem Prozentsatz im Verhältnis zum Kontrollmittel (Henseleit U, Rosenbach T, Kolde G :"Induction of apoptosis in human HaCaT keratinocytes."; Archiv. Dermatol. Res. (288) 11, 676-683, 1996) angegeben.

**Tab. 2a:**

| **Anti-Apoptose Aktivität von Vigna aconitifolia extract an Humanfibroblasten im Vergleich zu extraktfreiem medium (Mittelwert von 4 Assays in dreifacher Bestimmung)** | | |
|---|---|---|
| **Konzentration (% Gew/Vol.)** | **Zellanzahl** | **Level an cytoplasmischen DNA-Fragmenten** |
| Control | 100 | 100 |
| 0.05 | 105 | 78 |
| 0.1 | 106 | 74 |

**Tab. 2b:**

| **Anti-Apoptose Aktivität von Vigna aconitifolia extract an Humankeratocyten im Vergleich zu extraktfreiem medium (Mittelwert von 4 Assays in dreifacher Bestimmung)** | | |
|---|---|---|
| **Konzentration (% Gew/Vol.)** | **Zellanzahl** | **Level an cytoplasmischen DNA-Fragmenten** |
| Control | 100 | 100 |
| 0.05 | 102 | 39 |
| 0.1 | 105 | 46 |

Die Ergebnisse in denTabellen 2a und 2b zeigen eine deutliche Verringerung des Apoptose-Levels für Keratinocyten und Fibroblasten bei Behandlung mit Vigna aconitifolia-Proteinextrakten. Bei den Keratinocyten steigt das Zellwachstum merklich, während die Fibroblasten eine extreme Verbesserung des Zellmetabolismus aufweisen.

Die Extrakte der Vigna aconitifolia aus der vorliegenden Erfindung haben beachtliche Fähigkeiten bewiesen, den Gehalt der Apoptosen zu reduzieren, die in einen Nährboden menschlicher Zellen induziert wurden, die einen Wachstumsfaktor entbehren, was die hohen Fähigkeiten dieser Extrakte erklärt das Altern von Gewebe durch eine "growth-factor-like"-Wirkung (Typ Wachstumsfaktor) zu bekämpfen. Es zeichnet die Extrakte zum Einsatz als Anti-Ageing Wirkstoff besonders aus.

### 3. Nachweis der Apoptose und Bestimmung des Gehaltes an apoptotischen Zellen - ex-vivo

Auch die TUNEL-Technik (Tdt-mediated UTP nick end labelling, Boehringer), die für die Bestimmung apoptotischer Kerne eingesetzt wird, eignet sich über die Detektion von strukturellen Änderungen in den Zellkernen zur Bestimmung des Apoptoselevels. Die durch die Antikörper hervorgerufenen Reaktionen müssen zur Bestimmung des Apoptoselevels quantifiziert werden.

In einer weiteren Technik bedient man sich einer immunohistochemischen Technik spezieller Antikörper gegen Ki67, einem Marker für die mitotische Aktivität der Zellen (Seigneurin D., Guillaud Ph - L'antigène Ki 67, marqueur du cycle cellulaire et de la prolifération tumorale, Pathol. Biol., 39, 10, 1020-1028, 1991).

Zur optischen Quantifizierung nutzt man in der Regel entweder ein Photonenmikroskop (AEC - Amino Ethyl Carbazole - Reaktion) oder ein CLSM-Mikroskop (confocal laser scanning microscope). Die damit aufgenommenen Bilder werden durch Bildanalyse quantifiziert. Die Intensität der Reaktion wird dabei durch einen Koeffizienten ausgedrückt, der proportional zur untersuchten Fläche ist.

### Zubereitung der menschlichen Zellen

Die Apoptose wurde ausgelöst durch den Entzug von Wachstumsfaktoren.
Menschliche Hautexplantate aus chirurgischen Eingriffen wurden in Hanks Medium mit 10 Gew.% DMEM (DMEM = Dulbecco Minimum Essential Medium) kultiviert.
Zwei Anwendungen einer Creme (Beispiel XX) wurden in einer Menge von 2 mg/cm² am ersten und dritten Tag aufgetragen. Danach erfolgte die Inkubation des Explantates bei 37°C mit 5% CO2-Athmosphäre über 7 Tage.

Nach erfolgter histologischer Aufarbeitung wurde die TUNEL-Technologie für epidermale vertikale Schnitte eingesetzt und Mitosestudien mit Ki67 an horizontalen Schnitten.

**Tab. 3a:**

| **Anti-Apoptose Aktivität von Vigna aconitifolia Extract behandelter Humanepidermis - Mitotische Aktivität (Ki67) im ex vivo - Versuch (Mittelwert von 5 Versuchen)** | |
|---|---|
| | Anzahl aktiver mitotischer Zellkerne / Fläche Epidermis |
| Kontrolle vor Beginn der Bestimmung (D0) - ohne Apoptose | 841.15 |
| Kontrolle ohne Behandlung D7* - mit Apoptose | 157.95 |
| Placebo Creme D7* | 120.9 |
| Creme mit Vigna aconitifolia extract 1% D7* | 175.9 |
| Creme mit Vigna aconitifolia extract 3% D7* | 221.25 |

| | |
|---|---|
| *: nach Inkubation in Hanks Medium mit 10% DMEM über 7 Tage | |

**Tab. 3b:**

| **Anti-Apoptose Aktivität von Vigna aconitifolia Extract behandelter Humanepidermis - Apoptose (TUNEL)- Bestimmung im ex vivo - Versuch (Mittelwert von 5 Versuchen)** | |
|---|---|
| | Anzahl apoptotischer Zellen / Fläche Epidermis |
| Kontrolle vor Beginn der Bestimmung (D0) - ohne Apoptose | 87.3 |
| Kontrolle ohne Behandlung D7* - mit Apoptose | 89.5 |
| Placebo Creme D7* | 99.1 |
| Creme mit Vigna aconitifolia extract 1% D7* | 13.5 |
| Creme mit Vigna aconitifolia extract 3% D7* | nicht detektierbar |

| | |
|---|---|
| *: nach Inkubation in Hanks Medium mit 10% DMEM über 7 Tage | |

Der Vergleich Kontrolle D0 und D7 gilt zur Überprüfung der Methode und zeigt, dass ein deutliches Apoptose gesteuertes Absterben der Zellen durch die Methoden zu detektieren ist.
Jedoch wurde bei Anwendung von Zubereitungen, die Vigna aconitifolia enthalten, eine deutliche Erhöhung der Mitoseaktivität und eine Verringerung der Apoptoseaktivität beobachtet.
Es läßt sich daraus ein starker Anti-Aging-Effekt ableiten.

### 4. Test zur Epidermalen Zellerneuerungsaktivität

### Bestimmung der Stimulation der epidermalen Zellerneuerung in vivo im Dihydroxyacetone - Test (DHA) - Test

Es wurden vergleichende Versuche zur Bestimmung der Aktivität zur beschleunigten epidermalen Zellerneuerung mit 5 Emulsionen enthaltend 0,2 Gew. % oder 0,5 Gew.% Vitamin-A-palmitat, 3 Gew.% oder 5 Gew.% VITOPTINE® (Extrakt der Vigna aconitifolia, Lab. Sero, Pulnoy) versus Placebo in-vivo durchgeführt.

| Zusammensetzung der Emulsionen: | |
|---|---|
| PhaseA | |
| Cetearyl alcohol and Ceteareth-20 | 5 |
| Glycreyl stearate and PEG-100 stearate | 3.5 |
| Glyceryl stearate | 2.5 |
| Cetearyl alcohol | 2 |
| Octyldodecanol | 5 |
| Paraffin | 3 |

| Phase B | |
|---|---|
| LS Stabilisator (Mehtylparaben, 58.2% - Dipotassium EDTA, 11.2%) : | 30.6% - Hexamidine diisethionate, 0.3 |
| Aqua conservata | ad 100,0 |
| | |

| Phase C | |
|---|---|
| Wirkstoffe | |

Phase A und Phase B werden auf 75°C erwärmt. Die wasserphase wird der Fettphase bei ständigem Rühren zugefügt und durch weiteres Rühren bis 60°C abgekühlt. Danach wird mit dem Ultraturrax 2 min. homogenisiert.
Schließlich wird der Wirkstoff unter Rühren zugefügt und bis zur Raumtemperatur abgekühlt.

### Studiendurchführung

Die Studie wurde als Doppelblindstudie an 12 weiblichen Probanden im Alter von 18 bis 55 Jahren durchgeführt.
Als Meßparameter diente die Intensität der Braunfärbung der Haut nach Anfärbung durch Dihydroxyaceton. Die abnehmende Braunfärbungsintensität ist ein Maß für die Erneuerung des Stratum Corneums und die Geschwindigkeit des epidermalen Zellerneuerungscyclus.

Markiert wurden 8 Bereiche (in Zweiergruppen) auf der Innenseite des unteren Vorderarms.
- Kontrollbereich nicht behandelt und nicht angefärbt durch DHA,
- Bereich behandelt mit Placebo Creme
- Bereich behandelt mit Creme enthaltend Vitamin-A-palmitat
- Bereich behandelt mit Creme enthaltend VITOPTINE
Täglich erfolgte eine standardisierte und randomisierte Vorbehandlung mit 3 mg/cm² einer Zubereitung über 7 Tage.
Nach Beendigung der Vorbehandlung wurde nach einem okklusiven standardisierten Verfahren am Tag 8 DHA in Form einer Bräunungsgesichts-und Körperlotion enthaltend 5 Gew.% DHA auf Kontroll- und behandelte Bereiche aufgetragen.
Am Tag 9 wurden die Okklusivpflaster entfernt. Es folgte dann an 6 weiteren Tagen wiederum die Behandlung entsprechend Tag 1 bis 7.
Die Farbintensität der Haut wurde unmittelbar nach der Pflasterentfernung und 6 Tage später durch colorimetrische Messungen (Minolta chromameter) über Lumineszenz bestimmt.

### Ergebnisse

**Tab. 4:**

| **Epidermale Zellerneuerung im in-vivo Test an 12 Probanden - Vergleich von Vitamin-A-palmitat und Vigna aconitifolia Extrakt gegen Placebo** | | | | | | | |
|---|---|---|---|---|---|---|---|
| Parameter | DHA - Pigmentierung Tag 9 (D9) | D15 | | | | | |
| | | Mittelwert Kontrollen | Mittelwert Placebocreme | Creme mit Vitamin-A- | | Creme mit Vigna aconitifolia Proteinextrakt | |
| | | | | 0,2 % | 0,5 % | 3 % | 5 % |
| Luminescence (AU) | 58,65 | 61,33 | 62,13 | 62,18 | 62,44 | 62,72 | 62,79 |
| Entwicklung Luminescence (D15-D9) | | 2,68 | 3,48 | 3,53 | 3,79 | 4,07 | 4,14 |
| Entwicklung Luminescence (D15-D9) | | | 30 | 32 | 41 | **52** | **54** |
| % referring to control area | | | (ns) | (ns) | (ns) | **(*)** | **(**)** |
| D9: nach Entfernung der Pflaster | | | | | | | |
| D15 : 6 Tage nach Entfernung der Pflaster | | | | | | | |
| AU : Arbitrary Unit | | | | | | | |
| Nach Wilcoxon T-Test (ns) nicht signifikant | | | | | | | |

| | | | | | | | |
|---|---|---|---|---|---|---|---|
| (*)p=0,05 | | | | | | | |
| (**) p = 0,04 | | | | | | | |

Die erste Berechnung (D15 - D9) erlaubte eine Bewertung der Entwicklung zwischen Tag 9 (Entfernung der Okklusivpflaster) und Tag 15 (6 Tage nach Entfernung der Okklusivpflaster).
Diese Entwicklung ist representativ für den epidermalen Zellerneuerungscyclus. Je größer der Wert ist, desto höher ist die epidermale Zellemeuerung.

### Zusammenfassung

Die vergleichenden Untersuchungen am Menschen zeigen eine wesentlich höhere epidermale Zellerneuerungsrate bei Behandlung mit Emulsionen, enthaltend 3 Gew. % oder 5 Gew. % des Extraktes von Vigna aconitifolia Samen gegenüber einer Behandlung mit Emulsionen enthaltend 0,2 Gew. oder 0,5 Gew.% Vitamin-A-palmitat.

### Gewerbliche Anwendung

Gegenstand der vorliegenden Erfindung sind ebenfalls kosmetische oder dermopharmazeutische Zusammensetzungen für die lokale Anwendung auf der Haut, den Epithelanhanggebilden und/oder den Schleimhäuten, die als Wirkstoff alleine oder in Verbindung mit mindestens einem anderen Wirkstoff, mindestens eine Proteinfraktion enthält, die aus Vigna aconitifolia-Samen extrahiert wurde.

Diese kosmetische Zusammensetzung kann als einzigen Wirkstoff oder mit mindestens einem anderen Wirkstoff verbunden, mindestens einen Extrakt des vorab genannten Typs enthalten, der verwendet wird, um mindestens eine der besonderen biologischen Wirkungen zu erzeugen, die vorab beschrieben wurden oder sogar mehrere dieser Wirkungen als Kombination.

Die kosmetische oder dermopharmazeutische Zusammensetzung der vorliegenden Erfindung kann zwischen 0,001 und 30 Gew. %, vorzugsweise zwischen 0,1 und 20 Gew. % und besonders bevorzugt zwischen 0,2 und 10 Gew. % eines Proteinhaltigen Extraktes enthalten, der aus Vigna aconitifolia-Samen extrahiert wurde (die Extrakte wurden durch eines der vorab erwähnten Verfahren gewonnen). Der Extrakt kann in geeignete kosmetische Vektoren, wie zum Beispiel Liposome, Makro-, Mikro- und Nanokapseln, Makro-, Mikro und Nanopartikel und andere analoge und bekannte Formen eingearbeitet werden. Die einzusetzende Konzentration der Vigna aconitifolia - Proteinextrakte in den Zubereitungen kann aufgrund der hohen Aktivität der Vigna aconitifolia gegenüber Proteinextraktgehalten anderer Vigna-Arten herabgesetzt werden.

Die vorab erwähnten Extrakte können für Anwendungen zur Pflege und Hygiene der Haut verwendet werden (Erzeugnisse für Gesicht und Körper, Tag- oder Nachtkosmetika, Sonnenschutzmittel, kräftigende, regenerierende Erzeugnisse, Anti-Faltenkosmetika, Schlankheitskuren-mittel, Mittel gegen Alterungsprozesse) in Form von Zubereitungen, wie zum Beispiel Lotionen oder Shampoos, Cremes, Schaummittel, Seifen, Stäbchen, Gele, Hydrogele, Sprühmittel, Emulsionen, Schutzmittel, reparierende, weichmachende, filmbildende und photoschützende Mittel ; Erzeugnisse für Dauerwellen und zur Haarfärbung.

Die vorab erwähnten Extrakte können zur Herstellung kosmetischer und/oder dermopharmazeutischer Mittel, wie beispielsweise Haarshampoos, Haarlotionen, Schaumbäder, Duschbäder, Cremes, Gele, Lotionen, alkoholische und wäßriglalkoholische Lösungen, Emulsionen, Wachs/ Fett-Massen, Stiftpräparaten, Pudern oder Salben, vornehmlich jedoch Erzeugnisse für Gesicht und Körper, Tagoder Nachtkosmetika, Sonnenschutzmittel, kräftigende, regenerierende Erzeugnisse, Anti-Faltenkosmetika, Schlankheitskurenmittel und Mittel gegen Alterungsprozesse dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale undloder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
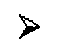 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
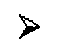 Alkyl- und/oder Alkenyloligoglykoside mit 8 bis 22 Kohlenstoffatomen im Alk(en)ylrest und deren ethoxylierte Analoga;
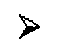 Anlagerungsprodukte von 1 bis 15 Mol Ethylenoxid an Ricinusöl und/oder gehärtetes Ricinusöl;
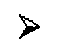 Anlagerungsprodukte von 15 bis 60 Mol Ethylenoxid an Ricinusöl und/oder gehärtetes Ricinusöl;
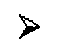 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
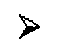 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
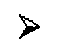 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
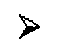 Mono-, Di- und Trialkylphosphate sowie Mono-, Di- und/oder Tri-PEG-alkylphosphate und deren Salze;
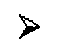 Wollwachsalkohole;
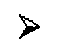 Polysiloxan-Polyalkyl-Polyether-Copolymere bzw. entsprechende Derivate;
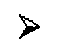 Block-Copolymere z.B. Polyethylenglycol-30 Dipolyhydroxystearate;
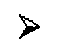 Polymeremulgatoren, z.B. Pemulen-Typen (TR-1,TR-2) von Goodrich;
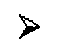 Polyalkylenglycole sowie
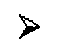 Glycerincarbonat.

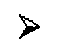 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

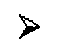 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

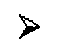 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

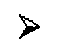 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

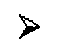 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

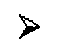 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

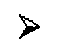 Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyloder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ lsobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
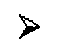 3-Benzylidencampher bzw. 3-Benzylidennorcampher und dessen Derivate, z.B. 3-(4-Methylbenzyliden)campher wie in der **EP 0693471 B1** beschrieben;
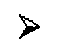 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
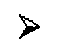 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
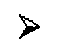 Ester der Salicylsäure, vorzugsweise Salicylsäure-2-ethylhexylester, Salicylsäure-4-isopropylbenzylester, Salicylsäurehomomenthylester;
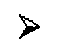 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
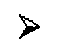 Ester der Benzalmalonsäure, vorzugsweise 4-Methoxybenzmalonsäuredi-2-ethylhexyl-ester;
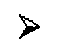 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
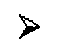 Propan-1,3-dione, wie z.B. 1-(4-tert.Butylphenyl)-3-(4'methoxyphenyl)propan-1,3-dion;
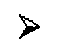 Ketotricyclo(5.2.1.0)decan-Derivate, wie in der **EP 0694521 B1** beschrieben.

### Als wasserlösliche Substanzen kommen in Frage:

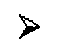 2-Phenylbenzimidazol-5-sulfonsäure und deren Alkali-, Erdalkali-, Ammonium-, Alkylammonium-, Alkanolammonium- und Glucammoniumsalze;
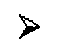 Sulfonsäurederivate von Benzophenonen, vorzugsweise 2-Hydroxy-4-methoxybenzophenon-5-sulfonsäure und ihre Salze;
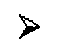 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, weitere Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

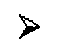 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

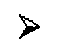 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

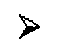 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

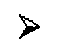 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
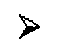 adstringierende Wirkstoffe,
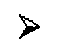 Ölkomponenten,
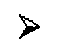 nichtionische Emulgatoren,
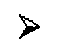 Coemulgatoren,
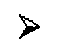 Konsistenzgeber,
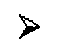 Hilfsstoffe wie z. B. Verdicker oder Komplexierungsmittel und/oder
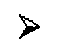 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
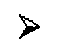 entzündungshemmende, hautschützende oder wohlriechende ätherische Öle,
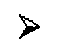 synthetische hautschützende Wirkstoffe und/oder
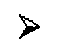 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
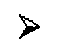 Glycerin;
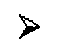 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
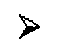 technische Oligoglyceringemische mit einem Eigenkondensationsgrad von 1,5 bis 10 wie etwa technische Diglyceringemische mit einem Diglyceringehalt von 40 bis 50 Gew.-%;
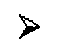 Methyolverbindungen, wie insbesondere Trimethylolethan, Trimethylolpropan, Trimethylolbutan, Pentaerythrit und Dipentaerythrit;
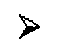 Niedrigalkylglucoside, insbesondere solche mit 1 bis 8 Kohlenstoffen im Alkylrest, wie beispielsweise Methyl- und Butylglucosid;
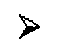 Zuckeralkohole mit 5 bis 12 Kohlenstoffatomen, wie beispielsweise Sorbit oder Mannit,
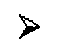 Zucker mit 5 bis 12 Kohlenstoffatomen, wie beispielsweise Glucose oder Saccharose;
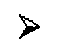 Aminozucker, wie beispielsweise Glucamin;
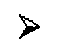 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Die Herstellung der Mittel kann durch übliche Kalt - oder Heißprozesse erfolgen; vorzugsweise arbeitet man nach der Phaseninversionstemperatur-Methode.

### Beispiele zur Gewinnung von Proteinextrakten

### I) ZUBEREITUNG DER PROTEINEXTRAKTE DER VIGNA ACONITIFOLIA-SAMEN

Die Zubereitung der Proteine erfolgt nach den bereits in der FR 2796839 A1 für Vigna trilobata beschriebenenen Methoden durch die herkömmlichen Techniken zur Extraktion von Pflanzenproteinen, der Zubereitung von Konzentraten oder Proteinisolaten oder durch Reinigung (Ultrafiltrieren, Ionenaustausch-Chromatographie, Affinitätschroma-tographie, Ausfällung, Adsorption), die dem Fachmann auf dem Gebiet bekannt sind.
Aber vorzugsweise erfolgt die Extraktion mit Wasser oder einer wässrigen Lösung bei einem gegebenen pH-Wert (siehe Beispiel 6 und 7), eventuell durch einen Ultraschallerzeuger.

Als Beispiele zur Veranschaulichung, aber nicht zur Einschränkung werden nachstehend verschiedene Vorgänge zur Gewinnung und Zubereitung von Proteinextrakten ausgehend von zwei verschiedenen, abgeteilten Vigna aconitifolia-Samenmengen indischen Ursprungs beschrieben.

### BEISPIEL 1

In 1,5 Liter destilliertes Wasser werden 180 g Mehl gegeben, das durch Zerquetschen trockener Vigna aconitifolia-Samen gewonnen wurde. Nach 15-minutigem Rühren wird der pH der Lösung dem pH von 7,0 mit Natronlauge angepasst.
Die Extraktion wird 3 Stunden lang bei Raumtemperatur vorgenommen, indem der Extraktions-pH-Wert bei 7,0 gehalten wird. Nach 10-minutigem Zentrifugieren bei 4500 g wird der auf der Oberfläche schwimmende beige Stoff aufgefangen und danach auf 0,5 µm filtriert.

Der Extrakt kann durch herkömmliche Techniken, wie zum Beispiel Zerstäubung, Gefriertrocknen oder dergleichen entwässert werden. Nach dem Zerstäuben wies das pulverartige gewonnene Erzeugnis einen Proteingehalt (N x 6,25) von 47,0 % (Extrakt 1) auf.

### BEISPIEL 2

In 2,5 Liter destilliertes Wasser werden 300 g Mehl gegeben, das durch Zerquetschen von Vigna aconitifolia-Samen gewonnen wurde und die Lösung wird wie im Beispiel 1 verarbeitet. Man gewinnt 2,2 Liter beige Lösung. Der pH-Wert der Lösung wird mit Schwefelsäure auf pH 5 angeglichen und 30 Minuten unter Rühren gelassen. Die Lösung wird danach 30 Minuten bei 3500 g zentrifugiert: der Niederschlag und die an der Oberfläche schwimmende Materie werden aufgefangen. Der Niederschlag wird als Lösung in ein Wasservolumen gegeben, das 20 % des Volumens vor dem Niederschlag entspricht. Der pH-Wert der Lösung wird mit NaOH angeglichen, bis er sich bei 7,2 stabilisiert.
Die Lösung wird wieder zentrifugiert, um die unlöslichen Stoffe abzusondern. Man gewinnt 600 ml Lösung eines 3,8 %igen trockenen Extrakts, der durch Zerstäubung entwässert wird.

Nach der Zerstäubung weist das gewonnene pulverartige Erzeugnis einen Proteingehalt (N x 6,25) von 84,2 % (Extrakt 2) auf.

### BEISPIEL 3

Die an der Oberfläche schwimmenden, nach dem Niederschlag der Proteine bei pH 4,5 nach dem Beispiel 2 gewonnenen Stoffe werden auf 0,5 µm und dann auf 0,22 µm gefiltert : die klaren, gewonnenen Lösungen werden durch Zerstäubung entwässert.
Das gewonnene Pulver weist eine Proteingehalt von 17 % bis 21 % auf.

### BEISPIEL 4

250 ml roher Extrakt (pH 7,5), der nach Beispiel 1 zubereitet wurde, werden in eine Ultrafilterungszelle des Typs Amicon 8200 gegeben, die mit einer 100 000 Da-Ultrafilterungsmembran (Ref. YM100, Durchmesser 6 cm) versehen ist. Die Lösung wird bis auf 55 ml konzentriert (Druck der Druckluft 3 bar). Permeat P1 und Retentat R1 werden aufgefangen.
Dem Retentat werden 150 ml destilliertes Wasser hinzugefügt und die Lösung wird noch einmal bis auf 50 ml konzentriert (Retentat R2). Retentat R2 wird durch Gefriertrocknung entwässert : man gewinnt eine Fraktion mit einem 84,5 %igen Proteingehalt (N x 6,25).

### BEISPIEL 5

Ein Proteinkonzentrat wird nach Beispiel 2 ausgehend von 350 g Samen zubereitet, wobei die anfängliche Extraktion in einem Verhältnis Mehl/Lösungsmittel von 1/15 vorgenommen wurde. Der Niederschlag wird bei pH 7,5 in 1,5 Liter destilliertem Wasser aufgelöst. Man gewinnt am Ende 500 ml Proteinkonzentratlösung mit 60,5 g/l an Proteinen (N x 6,25).
Die Proteine werden bei pH zwischen 7,5 und 8,5 mit einer Alkalinprotease hydrolysiert (2,5 % im Verhältnis zu den Proteinen der Lösung). Die Hydrolyse wird 2 Stunden lang bei optimierter Temperatur und optimiertem pH-Wert des verwendeten Enzyms vorgenommen, die optimierten Werte sind dem Fachmann bekannt. Das Enzym wird durch Erhitzen auf 100° C während mindestens 10 Minuten inaktiviert. Nach der Abkühlung auf Raumtemperatur wird die Lösung zentrifugiert und dann bis auf 0,22 µm gefiltert. Man gewinnt 450 ml dunkles, reines Filtrat bei 6,53 % Trockenextrakt (Proteine 45,2 g/l).

Nach der Zerstäubung weist das gewonnene Pulver eine Proteingehalt von 72,0 % auf (Extrakt 3) auf,
Die Analyse durch Gelpermeation auf einer Säule des Typs Superose 12 HR der durch diese verschiedenen Methoden extrahierten Fraktionen ermöglicht mindestens 10 Proteinfraktionen zu kennzeichnen.
Dabei liegen 80 % < 5000 Da und 20 % > 5000Da und < 50 000 Da.
Die Bestandteile des Hydrolysats, das nach dem vorhergegangenen Beispiel 5 ausgeführt wurde, weisen ein durchschnittliches Molekulargewicht von 3 500 Da auf.

Die durch die beschriebenen Verfahrensbeispiele gewonnenen Extrakte sind direkt in flüssiger Form verwendbar oder nach dem Trocknen nach den herkömmlichen Entwässerungstechniken (Zerstäubung, Gefriertrocknung). Die gewonnenen Proteinfraktionen können entweder in ihrer ursprünglichen Form, ohne eine Veränderung der Strukturen oder in der Form einer oder mehrerer natürlichen Verbindung/en von mindestens zwei oder allen extrahierten Fraktionen unterschiedlichen sichtbaren Molekulargewichte verwendet werden, die verschiedenen Chromatogrammpeaks entsprechen, die auf den beiliegenden Zeichnungen dargestellt sind und die sich auf natürliche Weise in den Samen (totaler oder teilweiser Proteinextrakt) oder in isolierter Form befinden.

Die Proteinfraktionen können in Zusammensetzungen in ihrer durch irgendeine der folgenden Bearbeitungen veränderten oder funktionalisierten Form verwendet werden :
- die Polymerisation der ursprünglichen Proteine;
- die chemische Hydrolyse der ursprünglichen Proteine;
- die enzymatische Hydrolyse der ursprünglichen Proteine durch Proteasen tierischen, pflanzlichen, mikrobiellen oder fungischen Ursprungs : Pepsin, Trypsin, Chymotrypsin, Papain, Pronase, Bromelaine, Endoproteinase, Thermitase, Proteasen des Bacillus subtillis, Aspergillus niger, Aspergillus aryzae (Subtilisin, Alkalase, Neutrase);
- mikrobische Veränderung mit der Verwendung von Proteinen von Vigna aconitifolia als Fermentationssubstrat durch verschiedene Mikroorganismen, wie zum Beispiel Hefesorten (Saccharomyces - Hefepilze), Schimmmelpilze (Aspergillus), Bakterien, (Bazillus und dergleichen);
- chemische oder enzymatische Funktionalisierung durch Verfahren wie zum Beispiel der Entzug des Stärkemehls, Succinilisierung oder Phosphorylisierung ;
- Quaternisierung ;
- Pfropfung der saccharidischen oder lipidischen Moleküle oder jegliche andere chemische Veränderung durch Pfropfung.

### BEISPIEL 6 - bevorzugstes Herstellungsverfahren

Vorzugsweise werden die erfindungsgemäßen Extrakte durch Extraktion der Samen mit Wasser bei 30°C bis 70°C, vorzugsweise 35°C bis 60°C und besonders bevorzugt 40°C bis 50°C über 1 bis 6, vorzugsweise 2 bis 4 Stunden extrahiert.
Rückstände und Solvens werden getrennt und 0,5 bis 3 Stunden, vorzugsweise 1 bis 2 Stunden bei 70 bis 90°C, vorzugsweise 75 bis 85°C erhitzt. Nach pH-Einstellung auf pH 5 bis 7, vorzugsweise pH 5,5 bis 6,5 wird zentrifugiert und filtriert.
Man erhält den Extrakt mit einem Stickstoffgehalt von mindestens 0,4, vorzugsweise mindestens 0,5 und besonders bevorzugt über 0,6 Gew. % mit einer Molekulargewichtsverteilung von : 80 % < 5000 Da und 20 % > 5000Da und < 50 000 Da (Molekulargewichtsbestimmung mit Superose® 12 HR, FPLC, Pharmacia).
Zu diesem Extrakt können weitere Hilfsstoffe wie Konservierungsmittel, Antioxidantien und Polyole zugefügt werden.

### BEISPIEL 7

### Herstellung des Produktes VITOPTINE® (Laboratoires Sérobiologiques) mit Vigna aconitifolia-Extrakt

### Herstellung

50 kg Samen der Vigna aconitifolia werden mit 200 kg - 300 kg Wasser bei 40 bis 50°C über 3 bis 4 Stunden extrahiert. Rückstände und Solvens werden getrennt und 1 Stunde bei 80°C erhitzt. Nach pH-Einstellung auf pH6 wird zentrifugiert (3500 g (75 Kg/h) und filtriert. Man erhält den Extrakt mit einem Stickstoffgehalt von 0,5 bis 1 Gew. %, zu dem die Komponenten Wasser, Glycerin, Natriumcitrat und Konservierungsmittel bei 60°C unter Rühren zugefügt werden. Es wird auf pH 6 eingestellt und erneut filtriert.

**Tab. 5:**

| **Zusammensetzung der proteinextrakthaltigen Formulierung der Vigna aconitifolia zum Einsatz in kosmetischen und/oder dermopharmazeutischen Zubereitungen (Mengenangaben in Gew. %)** | | | |
|---|---|---|---|
| **Bestandteile** | erfindungsgemäße Zusammensetzung | vorzugsweise | besonders bevorzugt |
| Vigna aconitifolia Samen Extrakt | 40 - 70 | 50 - 65 | 54 - 62 |
| Glycerin | 1 - 50 | 10 - 40 | 15 - 30 |
| Natriumcitrat | 0,01 - 3 | 0,1 - 2 | 0,5 - 1,5 |
| Konservierungsmittel (Chlorphenesin, Methylparaben) | q.s. | q.s. | q.s. |
| Wasser | ad 100,0 | ad 100,0 | ad 100,0 |

Die nach Beispiel 6 hergestellten Vigna aconitifolia - Extrakte eignen sich in Kombination mit den in Tabelle 5 beschriebenen Hilfsstoffen besonders zum Einsatz in kosmetischen und/oder dermopharmazeutischen Zubereitungen, da sie lagerstabile Konzentrate darstellen, die leicht in Formulierungen verarbeitet werden können und zu physiko-chemisch stabilen und dermatologisch sehr gut verträglichen Zubereitungen führen.

### Rezepturbeispiele

Als nicht einschränkende Beispiele werden nachstehend verschiedene Beispiele für die praktische Ausführung kosmetischer Zusammensetzungen nach der Erfindung beschrieben.

### BEISPIEL 1

Regenerationscreme - (dest. Wasser ad 100,0, Mengenangaben in Gew.%)

| Fettphase | |
|---|---|
| Ceteareth 25 | 2,00 |
| Ceteareth 6 und Stearylalkohol | 1,00 |
| Cetylalkohol | 4,00 |
| Glycolstearat | 4,00 |
| Vaseline | 5,00 |
| MCT- Triglyzeride, Miglyol | 5,00 |
| | |

| Wässrige Phase | |
|---|---|
| Glycerin | 10,00 |
| Vigna aconitifolia-Proteinextrakt nach Beispiel 1 der Verfahren | 2,00 |
| destilliertes Wasser | 8,50 |
| Elestab 4112 Konservierungsmittel (Laboratoires Sérobiol.) | 0,40 |
| Parfum | q.s. |

Die Fettphase wird bei 80° C aufgeschmolzen. Die wässrige Phase wird ebenfalls auf 80° C erhitzt und Elestab 4112 darin aufgelöst. Die Mutterlösung des Vigna-Extrakts Vitoptine® wird separat vorbereitet, die fette Phase in die wässrige Phase unter Turbinenrühren gegeben, und danach bei ungefähr 50° C Vitoptine® hinzugefügt. Danach wird mit dem Rühren bis zur Abkühlung fortgefahren.

### BEISPIEL 2

Creme für empfindliche Hauttypen und zur Behandlung von endzündlichen oder wunden Bereichen (dest. Wasser ad 100,0, Mengenangaben in Gew.%)

| Fettphase | |
|---|---|
| Glycolstearat | 14,00 |
| Octyl-Dodecanol | 6,00 |
| Dibutyl-Adipat | 6,00 |
| Ceteareth 12 | 1,50 |
| Ceteareth 20 | 1,50 |

| Wässrige Phase | |
|---|---|
| PVP (Polyvinylpyrrolidon) | 0,50 |
| Glycerin | 4,00 |
| Elestab 388 (Laboratoires Sérobiologiques) | 2,00 |
| Vigna aconitifolia nach Beispiel 2 der Verfahren | 3,00 |
| destilliertes Wasser | 9,00 |
| Parfum | 0,20 |

Die Fettphase wird bei 80° C aufgeschmolzen. Die wässrige Phase wird ebenfalls auf 80° C erhitzt und Elestab 388 und PVP darin aufgelöst. Vitoptine® wird separat vorbereitet, die fette Phase in die wässrige Phase unter Turbinenrühren gegeben, und danach bei ungefähr 50° Vitoptine® hinzugefügt. Danach wird mit dem Rühren bis zur Abkühlung fortgefahren.

In den Tabellen 5a und 5b sind weitere Vorschläge für Rezepturen mit Vigna aconitifolia- Extrakt VITOPTINE® (Laboratoires Sérobiologiques) beschrieben.

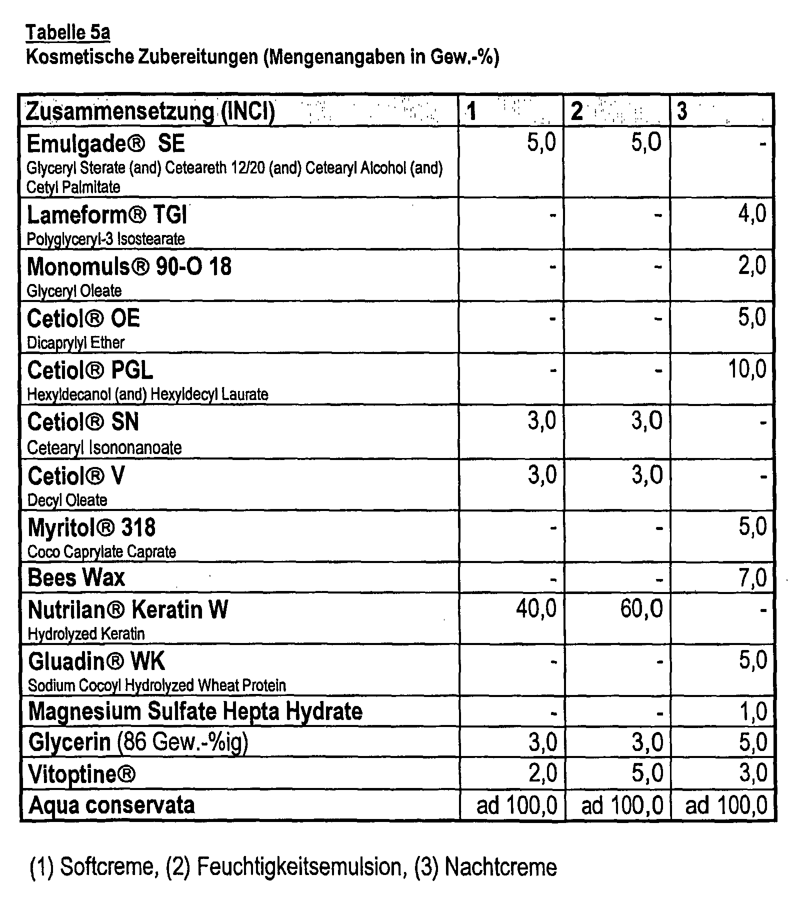

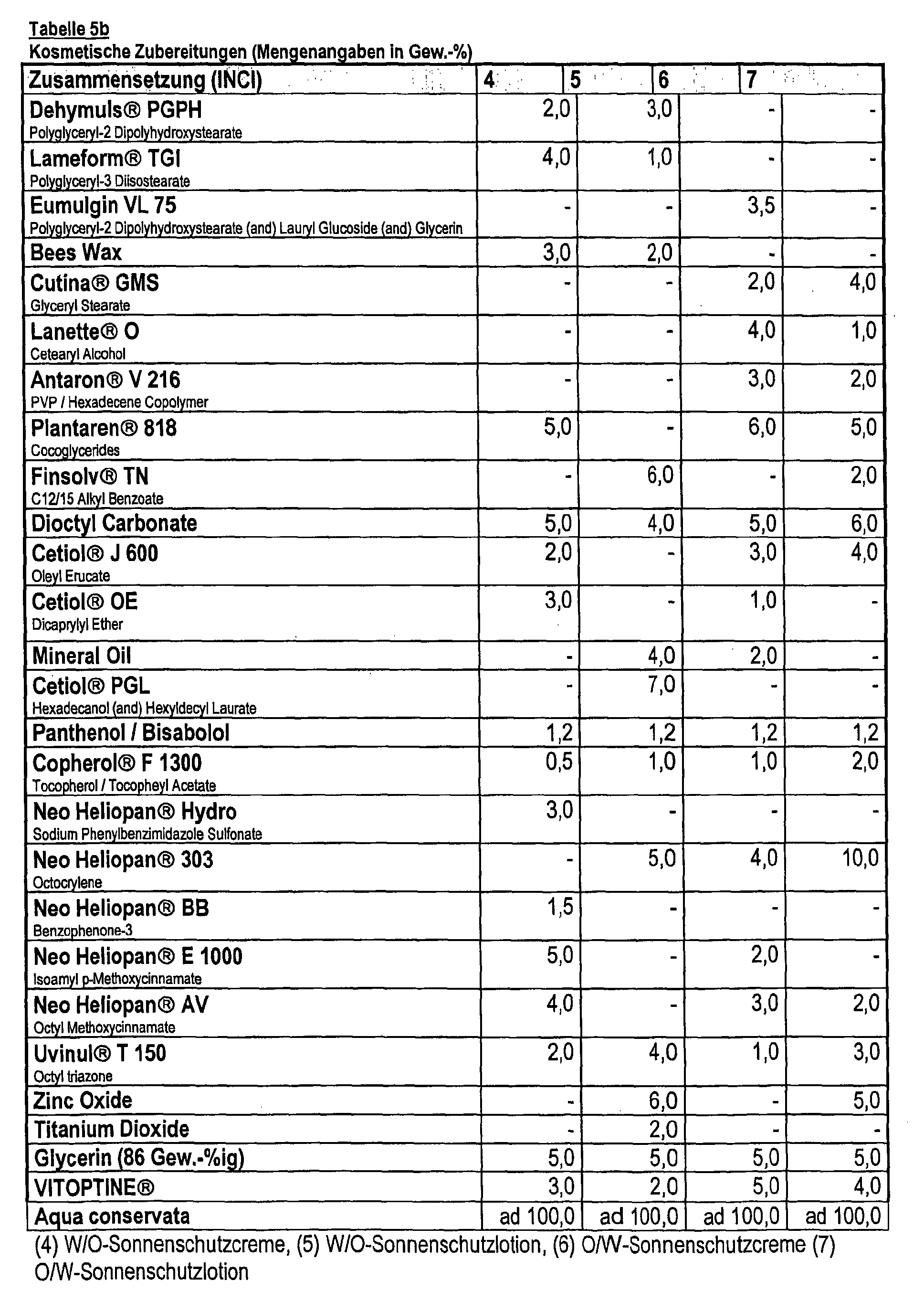

## Patentansprüche

1. Verwendung eines aus der Pflanze Vigna aconitifolia extrahierten proteinhaltigen Extraktes zur Herstellung kosmetischer und/oder dermopharmazeutischer Zubereitungen für die lokale Anwendung auf der Haut, den Epithelanhanggebilden und/oder den Schleimhäuten.

2. Verwendung nach Anspruch 1, **dadurch gekennzeichnet, dass** der Extrakt aus den Vigna aconitifolia-Samen als Wirkstoff eingesetzt wird, der Aktivitäten zur Stimulation des Wachstums und des zellularen Metabolismus, der Anti-Apoptose-Aktivitäten, der Anti-Entzündungs-Aktivitäten, der Anti-Ageing Aktivitäten und/oder der zellschützende Aktivitäten aufweist.

3. Verwendung nach einem der Ansprüche 1 und 2, **dadurch gekennzeichnet, dass** die Proteinfraktion/en des Extraktes durch Wasser oder eine Salzlösung bei einem gegebenen pH-Wert extrahiert wurde/n.

4. Verwendung nach irgendeinem der Ansprüche 1 bis 3, **dadurch gekennzeichnet, dass** die Protein-Fraktion/en als wässrige Lösung durch einen Ultraschallgenerator extrahiert wurde/n.

5. Verwendung nach einem der Ansprüche 3 und 4, **dadurch gekennzeichnet, dass** die Protein-Fraktion/en durch einen Vorgang gereinigt wird/werden, der in der von Ausfällen, Adsorption, lonenaustausch-Chromatographie, Affinitäts-Chromatographie und Ultrafiltration gebildeten Gruppe ausgewählt wird.

6. Verwendung nach irgendeinem der Ansprüche 1 bis 5, **dadurch gekennzeichnet, dass** die extrahierte Fraktion aus einer mit Proteasenhemmern angereicherten Fraktion besteht.

7. Verwendung nach irgendeinem der Ansprüche 1 bis 6, **dadurch gekennzeichnet, dass** die Proteinfraktion/en einen Wirkstoff bildet/n, der aus einem chemischen oder enzymatischen Hydrolysat besteht, das ausgehend von ursprünglichen Proteinen zubereitet wurde.

8. Verwendung nach irgendeinem der Ansprüche 1 bis 6, **dadurch gekennzeichnet, dass** die End-Proteinfraktion/en durch Polymerisation der ursprünglichen Proteine gewonnen wurde/n.

9. Verwendung nach irgendeinem der Ansprüche 1 bis 6, **dadurch gekennzeichnet, dass** die extrahierte/n Proteinfraktion/en chemisch durch Pfropfung verändert worden ist/sind.

10. Verwendung nach irgendeinem der Patentansprüche 1 bis 9, **dadurch gekennzeichnet, dass** die extrahierten Fraktionen die den Wirkstoff bilden, mindestens zwei Proteinfraktionen enthalten, deren sichtbare Molekulargewichte verschieden sind.

11. Verwendung nach irgendeinem der Ansprüche 1 bis 5, **dadurch gekennzeichnet, dass** der Extrakt aus einem totalen Extrakt besteht, der durch die gesamten extrahierbaren Proteinfraktionen gebildet wird, die von Natur aus in den Samen vorhanden sind.

12. Kosmetische und/oder dermopharmazeutische Zubereitungen für die lokale Applikation, enthaltend einen proteinhaltigen Extrakt der Pflanze Vigna aconitifolia.

13. Kosmetische und/oder dermopharmazeutische Zubereitungen für die lokale Applikation, enthaltend einen proteinhaltigen Extrakt aus den Samen der Pflanze Vigna aconitifolia.

14. Kosmetische oder dermopharmazeutische Zubereitungen nach den Ansprüchen 12 und/oder 13, **dadurch gekennzeichnet, dass** sie als Wirkstoff, der das Wachstum und den zellularen Metabolismus stimuliert, mindestens eine lösbare Proteinfraktion enthält, die aus Vigna aconitifolia-Samen extrahiert wurde.

15. Kosmetische oder dermopharmazeutische Zubereitungen nach mindestens einem der Ansprüche 12 bis 14, **dadurch gekennzeichnet, dass** sie 0,001 bis 30 Gew. % eines proteinhaltigen Extraktes, der aus Vigna aconitifolia extrahiert wurde, enthalten.

16. Kosmetische und/oder dermopharmazeutische Zubereitungen nach mindestens einem der Ansprüche 12 bis 15, **dadurch gekennzeichnet, dass** sie als Anti-Apoptose-Wirkstoff mindestens eine lösbare Proteinfraktion enthält, die aus Vigna aconitifolia-Samen extrahiert wurde.

17. Kosmetische und/oder dermopharmazeutische Zubereitungen nach irgendeinem der Ansprüche 12 bis 16, **dadurch gekennzeichnet, dass** sie als Wirkstoff zur vorbeugenden und heilenden Behandlung empfindlicher Hauttypen mindestens eine lösbare Proteinfraktion enthält, die aus Vigna aconitifolia-Samen extrahiert wurde.

18. Zubereitung enthaltend
a) 40 bis 70 Gew.% Extrakt des Samens von Vigna aconitifolia und
b) 10 bis 50 Gew. % Polyole

19. Verfahren zur Herstellung eines Vigna aconitifolia-Extraktes, dadurch charakterisiert, dass man
a) die Samen der Vigna aconitifolia mit Wasser extrahiert
b) Rückstände und Solvens trennt
c) nach pH-Einstellung auf pH 5 bis 7 zentrifugiert und filtriert und diesen Extrakt gegebenfalls mit weiteren Hilfsstoffen mischt.
